# EUROPÄISCHE PATENTSCHRIFT

(11) **EP 0 575 613 B1**
(45) Veröffentlichungstag und Bekanntmachung des Hinweises auf die Patenterteilung: **22.07.1998**
(21) Anmeldenummer: 92903883.4
(22) Anmeldetag: 10.01.1992
(51) Int. Cl.: A61K 31/195, A61K 9/20, A61K 47/38

(54) **VERWENDUNG DER AMINOSÄURE GLYCIN ZUR HERSTELLUNG VON ARZNEIMITTELN MIT ANTISTRESS-, STRESSSCHUTZ- UND NOOTROPER WIRKUNG**
USE OF THE AMINO ACID GLYCINE FOR THE PREPARATION OF A MEDICAMENT WITH ANTISTRESS-, STRESS-PREVENTIVE AND NOOTROPIC ACTION
UTILISATION DE L'AMINOACIDE GLYCINE POUR LA PREPARATION D'UN MEDICAMENT A ACTION ANTISTRESS, DE PREVENTION DU STRESS ET NOOTROPE

(43) Veröffentlichungstag der Anmeldung: 29.12.1993
(73) Patentinhaber: KOMISSAROVA, Irina Alexeevna, Moscow, 115304 (RU); GUDKOVA, Julia Vasilievna, Moscow 123298 (RU); SOLDATENKOVA, Tatyana Dmitrievna, Moscow, 109028 (RU); KONDRASHOVA, Tatyana Tikhonovna, Moscow, 129224 (RU); BURBENSKAYA, Natalya Mokhailovna, Moscovskaya obl., 140476 (RU)
(72) Erfinder: KOMISSAROVA, Irina Alexeevna, Moscow, 115304 (RU); GUDKOVA, Julia Vasilievna, Moscow 123298 (RU); SOLDATENKOVA, Tatyana Dmitrievna, Moscow, 109028 (RU); KONDRASHOVA, Tatyana Tikhonovna, Moscow, 129224 (RU); BURBENSKAYA, Natalya Mokhailovna, Moscovskaya obl., 140476 (RU)
(74) Vertreter: Hoffmann, Klaus, Dr. rer. nat.
(86) Internationale Anmeldenummer: RU9200005
(87) Internationale Veröffentlichungsnummer: WO9313764

(56) Entgegenhaltungen:
- EP-A- 0 124 091
- EP-A- 0 288 447
- FR-A- 2 437 834
- US-A- 4 438 144
- US-A- 4 539 315
- US-A- 4 562 205
- ARCH.GERONTOL.GERIATR. Bd. 5, Nr. 3 , Oktober 1986 Seiten 165 - 181 B.SALETU ET AL. 'Acute and subacute CNS effects of milacemide in elderly people: double-blind, placebo-controlled quantitative EEG and psychometric investigations'
- CLIN.NEUROPHARMACOLOGY Bd. 12, Nr. 5 , Oktober 1989 Seiten 416 - 424 R.B.ROSSE ET AL. 'Glycine Adjuvant Therapy to Conventional Neuroleptic Treatment in Schizophrenia: An Open-Label, Pilot Study'
- NEUROSCI.LETT. Bd. 101, Nr. 1 , 5. Juni 1989 Seiten 62 - 66 A.W.PROCTER ET AL. 'Loss of glycine-dependent radioligand binding to the N-methyl-D-aspartate-phencyclidine receptor complex in patients with Alzheimer's disease'
- ANN.N.Y.ACAD.SCI. Bd. 640 , 1991 Seiten 237 - 240 R.L.HERTING 'Milacemide and Other Drugs Active at Glutamate NMDA Receptors as Potential Treatment for Dementia'
- PHARMOCOL.BIOCHEM.BEHAV. Bd. 34, Nr. 4 , Dezember 1989 Seiten 823 - 828 G.E.HANDELMANN ET AL. 'Milacemide: a Glycine Prodrug, Enhances Performance of Learning Tasks in Normal and Amnestic Rodents'
- Referativny zhurnal "Khimia", Vol. 19, No. 22, 1991, VINITI, (Moscou), page 60, Abstract No. 22 0214 P.

## Beschreibung

### Gebiet der Technik

Die Erfindung bezieht sich auf die Medizin und betrifft insbesondere die Verwendung von Glycin zur Herstellung von Arzneimitteln mit Antistress-, Stressschutz- und nootroper Wirkung.

### Stand der Technik

Die Reaktion eines Menschen in einer Stresssituation ist meistenteils eine psychoemotionelle Spannung, die durch das Auftreten einer Erregung, einer Unruhe, einer Gedächtnisschwäche und einer Verschlechterung dem Aufmerksamkeit geleitet wird und sich in irrationalen, nicht adäquaten Verhaltensweisen, einer Verschlechterung und Störung der geistigen Arbeitsfähigkeit, der Reaktionsschnelligkeit, einer Vermehrung fehlerhafter Lösung u. dgl. zeigt. Ähnliche Störungen werden auch bei verschiedenen Erkrankungen (Neurose, neuroseähnlichen Zuständen, asthenoneurotischen Reaktionen u.a) beobachtet, und die Grösse dieser Störungen nimmt bei heftigen und chronischen Stressen zu.

Von besonderer Bedeutung ist daher die Suche und Schaffung von Präparaten, welche die Wirkung eines Stressfaktors ermässigen und verhindern und welche zugleich nootrope Eigenschaften aufweisen (eine Verbesserung der Aufmerksamkeit, des Gedächtnisses, der geistigen Tätigkeit und der Beständigkeit des Gehirnes geben aggressive Wirkungen).

Infolge dessen, dass die psycho-emotionelle Spannung eine Reaktion des Organismus auf verschiedene übermässige Erreger ist, bedient man sich zum Abbau dieser Spannung der Beruhigungsmittel, unter denen Tranquillizer aus der Benzdiazepin-Reihe sowie Gammaaminobuttersäure-Derivate (GAMK) wie Phenibut, Baklofen und Lyoresal, welche daneben eine nootrope Wirkung besitzem, allgemein bekannt und am häufigsten verwendbar sind. (M.D. Maschkowski. Arzneimittel, 1988, T.I, Verl. "Medizina", Moskau, S. 70 bis 79, 122 bis 123). Jedoch die Tranquillizer aus der Benzdiazepin-Reihe und die erwähnten GAMK-Derivate zeichnen sich durch ernsthafte Nebenwirkungen aus. Deren Einnahme bewirkt eine Schläfrigkeit, eine verzögerte Reaktion, einen Schwindel, eine Verschlechterung psychomotorischer Reaktionen, eine Störung der Gangart und der Monatsblutung und der Libido und ist für die Leute nicht angezeigt, deren Benehmen eine schnelle und genaue psychische Bewegungsreaktion voraussetzt. Hinzu kommt, dass durch eine langwierige Einnahme von Tranquillizers die geistige Tätigkeit verschlechtert wird und eine Toxikomanie entsteht.

Allgemein bekannt und anwendbar sind als nootrope Mittel eine Anzahl von GAMK-Derivaten, doch die letzteren weisen keine Antistress- bzw. Stressschutzwirkung auf (M.D. Maschkowski, Arzneimittel, 1988, T.I, Verl. "Medizina", Moskau, S. 117 und S. 123).

Bekannt ist der Einsatz von Glyzin als Bestandteil des Präparates Mega 3 Vitamins/Minerals der Fa. "Ortho-Biotics", welches man bei Stresszustanden und zur Verbesserung der Geistigen Tätigkeit benutzt. (Professional Ortho-Biotics, Catalogue 1989, CH, 1203, Geneve, Pharmacie des Charmilles, p. 18). Doch dieses Präparat enthält neben dem Glyzin über 34 weitere verschiedene Komponenten einschliesslich der Vitamine mit einer Allergiewirkung und ist durch hohe Selbstkosten gekennzeichnet. Darüber hinaus ist dessen Komponente Tryptophan, dessen Einsatz in pharmazeutischen Präparaten und als Zusatzstoff für Lebensmittel derzeit durch FDA (Dezember 1989) verboten wurde.

Aus der US-P 4,539,315 ist ferner eine analgetisch wirkende Mischung aus Acetylsalicilsäure und Glycin bekannt bei der die Acetylsalicilsäure durch den Anteil an Glycin weniger toxisch und besser resorbierbar ist. Eine eigene pharmakologische Wirkung des Glycins wurde nicht beschrieben.

Aus EP-A-0 124 091 ist eine Mischung aus Glycin, Sarcosin und N,N-Dimethylglycin bekannt, die einen synergistischen Effekt bei epileptischen Anfällen zeigt.

Aus EP-A-0 288 447 ist eine Mischung aus Taurin, L-Carnitin, Zn-Gluconat und Ca-Glycerinphosphat bekannt, welche auch Glycin oder andere Aminosäuren enthalten kann, und den Transport von Taurin und die Taurinabsorbtion der Nervenzellen fördert.

### Offenbarung der Erfindung

Die vorgeschlagene Verwendung ist neu und in der Fachliteratur nicht beschrieben. Der Erfindung liegt die Aufgabe zugrunde, ein neues Arzneimittel zu schaffen, das zugleich eine Antistress-, Stressschutz- und nootrope Wirkung aufweist, hoch aktiv und nicht toxisch ist und keine Nebenwirkungen nach sich zieht.

Diese Aufgabe ist dadurch gelöst worden, dass das anmeldungsgemässe Arzneimittel mit einem Wirkstoff und pharmazeutischem Träger erfindungsgemäss als Wirkstoff die Aminosäure Glyzin (Glykokoll, Aminoessigsäure) der Formel 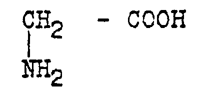 enthält.

Das erfindungsgemässe Arzneimittel weist eine hocheffiziente Antistress-, Stressschutz- und nootrope Wirkung auf und wird bei Vorbeugung und Behandlung von Neurosen und neuroseähnlichen Zuständen, Resterscheinungen von Schädel- und Hirntraumen und Neuroinfektionen, asthenovegetativen Störungen, dauernder Alkoholintoxifikation, Erscheinungen der Alkoholabstinenz sowie zur Verbesserung der geistigen Arbeitsfähigkeit eingesetzt.

Das Arzneimittel bietet sich als Pulver , gesättigte Lösung oder Tabletten an. Vorzugsweise benutzt man es als Unterzungentabletten mit dem Gehalt an dem Wirkstoff zwischen 0,1 und 0,2 g pro Tablette. Vorzugsweise enthält das Arzneimittel als pharmazeutischen Träger Methylcellulose in einer Menge von 0,5 bis 2 Gew.-%. Den Wirkstoff für das erfindungsgemässe Arzneimittel erhält man durch eine chemische Synthese, auf biotechnologische Weise oder aus Tiergeweben.

### Bevorzugte Ausführungsform der Erfindung

Der Wirkstoff des anmeldungsgemässen Arzneimittels ist eine aliphatische Aminosäure Glykol. Diese Aminosäure ist eine natürliche Verbindung, welche in Tiergeweben enthalten ist und welche einen pulverartigen, weissen Stoff ohne Geruch darstellt, der gut in Wasser und alkalischen Lösungen und schlecht in Ethylalkohol und Ester löslich ist.

Das erfindungsgemässe Arzneimittel wurde versuchsweise an Tieren und in der Klinik an Menschen getestet.

Die Antistress- und Stressschutzwirkung sowie die nootropen Effekte des vorgeschlagenen Arzneimittels untersuchte man an geschlechtsreifen Rattenmännchen mit je einem Gewicht zwischen 200 und 300 g sowie an nicht geschlechtsreifen Rattenmännchen mit einem Gewicht von je 30 bis 40 g und von 60 bis 80 g und an Mäusen mit je einem Gewicht zwischen 18 und 29 g.

Den Einfluss des erfindungsgemässen Arzneimittels auf das Verhalten von Tieren beim Test "Offenes Feld" untersuchte man nach einer akuten Stresswirkung an 20 Tage alten Rattenmännchen mit einem Gewicht von je 30 bis 40 g. Versuchs- und Kontrollrattenjunge wurden innerhalb einer Sekunde einem Elektroschock (elektrischer Fussboden mit 59 Hz und 35 V) ausgesetzt. Vor dem Elektroschock (20 min davor) wurde den Rattenjungen auf die Zungenwurzel das in Wasser aufgelöste Arzneimittel in einer Menge von 2 mg/kg Körpergewicht und der Kontrollgruppe nur noch die entsprechende Wassermenge gebracht. Nach Ablauf von 60 min untersuchte man das Verhalten der Tiere im Test "Offenes Feld". Daneben bediente man sich der Schätzungen des Verhaltens der 20 Tage alten Rattenjungen beim Test "Offenes Feld" als Kontrollergebnisse, denen 60 min vor dem Test nur noch Wasser eingeführt wurde. Die Testergebnisse sind in Tabelle 1 wiedergegeben.

**Tabelle 1**

| Einfluss des erfindungsgemässen Arzneimittels auf das Verhalten der Ratten im Test "Offenes Feld" nach akuter Stresswirkung | | | | | | |
|---|---|---|---|---|---|---|
| Tiergruppen | Verhaltensdaten M±m | | | | | |
| | L | r | A | S | W | D |
| Test (Elektroschock + Arzneimittel) | 45,04**± 13,46 | 1,0**± 0,28 | 0 | 1,0**± 0,01 | 0,4± 0,28 | 0,4**± 0,42 |
| 1. Kontrolle (Elektroschock ohne Arzneimittel) | 101,0*± 9,68 | 3,3± 0,48 | 0 | 11,0*± 2,74 | 1,2± 0,32 | 2,0± 0,48 |
| 2.Kontrolle (Ohne Elektroschock u. Arzneimittel) | 28,0± 12,98 | - | - | 1,7± 0,34 | 1,0± 0,34 | 1,0± 1,03 |
| Anmerkung: L- Läufe; r - rückgängige Bewegungen; A - Ausgänge; S - Stellungen; D - Defäkation; W - Waschen; | | | | | | |

| | | | | | | |
|---|---|---|---|---|---|---|
| * - Abweichung von der Kontrolle 2 ist wahrheitsgetreu, p < 0,05; | | | | | | |
| ** - Abweichung von der Kontrolle 1 ist wahrheitsgetreu, p < 0,05. | | | | | | |

Wie aus Tabelle 1 entnehmbar ist, weist des erfindungsgemässe, vorher eingeführte Arzneimittel eine Stressschutzwirkung auf, indem die durch den Stress verursachten Erregungserscheinungen sowohl in Bezug auf die Bewegungs- als auch in Bezug auf die emotionelle Erkenntnisaktivität im wesentlichen abgebaut werden.

Untersucht wurde die Stressschutzwirkung des erfindungsgemässen Arzneimittels beim Test "Elektrischer Fussboden", bei dem nach einem einfachen Elektroschock eines Paaree Mäusemännchen eine emotionelle (ein Piepsen), eine Bewegungs- (Sprünge) und eine aggressive (Schlägereien) Komponente der Reaktion erfasst wurden. Das erfindungsgemässe Arzneimittel, das 60 min vor dem Versuch eingeführt wurde, beeinflusste nicht die emotionelle Reaktion, verstärkte gewissermassen (um 1 bis 1,5 Punkte) die Bewegungskomponente, verringerte dafür die Zahl von Schlägereien und erhöhte um 5±0,8 V die Schwelle der Aggressionsreaktion.

Die Antistress- und Stressschutzwirkung des erfindungsgemässen Arzneimittels wurde an Rattenjungen mit einem Gewicht von je 30 bis 40 g und an erwachsenen Rattenmännchen mit einem Gewicht von je 200 bis 250 g unter Verwendung eines Modells des Elektroschocks untersucht. Für Versuchs- und Kontrollgruppen bediente man sich zur Bewertung der Auswirkungen des erfindungsgemässen Arzneimittels eines Testes, bei dem 15%iges Ethanol unter Bedingungen einer freien Wahl zwischen Ethanol und Wasser eingesetzt wurde, denn eine dauernde Stresswirkung wird durch eine höhere Alkoholmotivation und durch einen grösseren Ethanolverbrauch geleitet. Zur Auslösung eines chronischen Stresses benutzt man einen "elektrischen Fussboden" (50 Hz, 30 bis 40 V für Rattenjunge und 60 V für erwachsene Tiere). Bei Versuchs- und Kontrolltiergruppen wurde innerhalb von 7 Tagen der individuelle Tagesverbrauch an 15%igem Ethanol bei freier Wahl zwischen 15%igem Ethanol und Wasser ermittelt. Später unterwarf man die Rattenjungen innerhalb von 14 Tagen einem täglichen fünffachen Elektroschock der Pfoten bei einer Dauer von 1 s 5 min lang, die grossen Tiere einem zehnfachen mit einer Dauer von 1 s 10 min lang. 20 min vor der schmerzlichen Reizung wurde der Versuchsgruppe eine wässrige Lösung des erfindungsgemässen Arzneimittels in einer Menge mischen 1 und 2 mg/kg Körpergewicht auf die Zungenwurzel und der Kontrollgruppe die entsprechende Wassermenge gebracht. Im Verlaufe von 3 Wochen erfasste man bei freier Wahl zwischen 15%igem Ethanol und Wasser den individuellen Tagesverbrauch des 15%igen Ethanols: 2 Wochen bei einem dauerden Stress und der Einführung des erfindungsgemassen Arzneimittels und 1 Woche nach Verzicht auf den Elektroschock und auf das erfindungsgemässe Arzneimittel. Die erzielten Daten sind in Tabelle 2 zusammengestellt.

**Tabelle 2**

| Einfluss des erfindungsgemässen Arzneimittels bei dauerndem Stress auf den Tagesverbrauch des 15%igen Ethanol durch Ratten (in ml, M±m) | | | | |
|---|---|---|---|---|
| Tiergruppen | Untersuchungsstufen | | | |
| | 1. Woche | 2. Woche | 3. Woche | 4. Woche |
| Rattenjunge: | | | | |
| Versuch | 2,7±0,2 | 4,4±0,3 | 2,0±0,2 | 1,6±0,18 |
| Kontrolle | 2,5±0,2 | 8,0±0,4 | 6,7±0,3 | 3,3±0,3 |
| Aussagekraft der Versuche, p | - | < 0,05 | < 0,05 | < 0,05 |

| Erwachsene Tiere: | | | | |
|---|---|---|---|---|
| Versuch | 1,8±0,08 | 3,4±0,13 | 2,5±0,15 | 2,3±0,3 |
| Kontrolle | 1,9±0,09 | 7,1±0,96 | 8,4±0,39 | 5,9±0,2 |
| Aussagekraft der Versuche, p | - | < 0,05 | < 0,05 | < 0,05 |

Die Daten der Tabelle 2 sprechen dafür, dass durch das erfindungsgemässe Arzneimittel die Alkoholmotivation der Tiere abgebaut wird, wobei dessen Antistresswirkung sich nicht nach dem Alter der Tiere richtet und nach Einstellung der Einführung und nach Verzicht auf den Elektroschock immer noch feststellbar ist. Der Antistresseffekt des erfindungsgemässen Arzneimittels wirkte sich auch auf das Verhalten der Tiere aus. So zeigten beispielsweise die Versuchsrattengruppen gegenüber den Kontrollrattengruppen während der gesamten Versuchsdauer eine erheblich mindere Aggressivität und Erregungsfähigkeit.

Es ist allgemein bekannt, dass Manipulationen mit den Tieren (Entnahme aus dem Käfig, Einführung eines Stoffes einschliesslich des Wassers) als eigenartiger Stressfaktor auftreten und einen langwierigen Erregungszustand, eine Unruhe und eine Aggression mit sich bringen. Um die Abhängigkeit der Antistresswirkung des erfindungsgemässen Arzneimittels von der Art des Stresses klarzustellen, untersuchte man den Einfluss des erfindungsmässen Arzneimittels auf 20 Tage alte Rattenjunge, denen auf die Zungenwurzel das erfindungsgemässe Arzneimittel in einer Menge von 1 mg/kg Körpergewicht (Test) und die entsprechende Wassermenge (Kontrolle) unter Bedingungen einer freien Wahl zwischen 15%igem Ethanol und Wasser gebracht wurde. Die Testergebnisse sind in Tabelle 3 erkenntlich.

**Tabelle 3**

| Einfluss des erfindungsgemässen Arzneimittels auf den Tagesverbrach des 15%igen Äthanols durch Ratten bei dauerndem emotionellen Stress ( in mg, M±m) | | | | |
|---|---|---|---|---|
| Tiergruppen | Untersuchungsstufen | | | |
| | 1. Woche | 2. Woche | 3. Woche | 4. Woche |
| 1 | 2 | 3 | 4 | 5 |
| Versuch | 3,6±0,3 | 4,0±0,3 | 2,3±0,3 | 2,7±0,2 |
| Kontrolle | 3,6±0,3 | 6,6±0,3 | 6,7±0,3 | 5,0±0,3 |
| Aussagekraft der Versuche, p | - | < 0,05 | < 0,05 | < 0,05 |

Aus der Tabelle 3 ergibt es sich, dass durch tägliche Manipulationen mit den Tieren die Alkoholmotivation anwächst und dass durch die Einführung des erfindungsgemässen Arzneimittels diese im wesentlichen abgebaut wird. Demgemäss hängt die Antistresswirkung des erfindungsgemässen Arzneimittels nicht von der Stressart ab.

Um nunmehr die neurotropen Effekte des erfindungsgemässen Arzneimittels und die Mechanismen von dessen Wirkung herauszufinden, untersuchte man die Auswirkungen des Arzneimittels auf die neurophysiologischen Vorgänge. Versuche wurden in vivo an Mäusemännchen mit einem Gewicht von je 18 bis 22 g und an Rattenmännchen mit einem Gewicht von je 200 bes 250 g sowie in vitro an isolierten Organen wie Krummdarm eines Meerschweinchens und Samenbläschen einer Ratte angestellt.

Man untersuchte vergleichsweise den Einfluss des erfindungsgemässen Arzneimittels und das Phenibuts, also eines GAMK-Derivates, auf die orientierte Bewegungsaktivität der Mäuse bei automatisierter Erfassung deren Bewegungen. Hierbei führte man das erfindungsgemässe Arzneimittel und Phenibut als wässrige Lösungen intraabdominal und der Kontrolltiergruppe die entsprechende Wassermenge ein. Die Untersuchungsergebnisse sind in Tabelle 4 wiedergegeben.

**Tabelle 4**

| Einfluss des erfindungsgemässen Arzneimittels auf die Bewegungsaktivität von Mäusen | | | | | |
|---|---|---|---|---|---|
| Menge in mg/kg Körpergewicht d. Tieres | Art d.Einführung | Tierzahl einer Gruppe | Zahl d. Bewegungen,erfasst durch d. Registrierapparat nach 10 min (M±m) | | |
| | | | erfindungsgem. Arzneimittel | Phenibut | Aussagekraft d. Versuche, p |
| Kontrolle | intraabdominal | 20 | 657±96 | 269±8,4 | < 0,05 |
| 2 | -"- | 10 | 371±56 | | < 0,05 |
| 20 | -"- | 10 | 365±79 | | < 0,05 |
| 40 | -"- | 10 | 324±56 | | < 0,05 |
| 40 | über d. Magen | 10 | 439±70 | | < 0,05 |
| 80 | intraabdominal | 10 | 398±48 | | < 0,05 |
| 100 | -"- | 10 | | 434±4,4 | < 0,01 |
| 200 | -"- | 10 | 275±23 | 186±8,7 | < 0,01 |
| 500 | -"- | 10 | | 19±3,0 | < 0,01 |

Wie die Tabelle erkennen lässt, geht die Einführung des erfindungsgemässen Arzneimittels 20 min vor der Eintragung mit einem Rückgang der Bewegungsaktivität der Tiere einher, der Schwächungsgrad der orientierten Bewegungsreaktion hingegen ist von der Menge das erfindungsgemässen Arzneimittels nicht abhängig. Ausserdem weicht dis Wirkung des erfindungsgemässen Arzneimittels nach der Art seiner Gegenreaktion von der das Phenibuts ab

Man untersuchte auch die Einflussnahme das erfindungsgemässen Arzneimittels auf die Rektaltemperatur von Mäusen. Durch die Einführung des erfindungsgemässen Arzneimittels in Mengen von 2, 20, 40, 80 und 200 mg/kg Körpergewicht des Tieres blieb die Körpertemperatur unverändert, im Gegensatz zu den Tranquillizern, welche die Körpertemperatur des Tieres abnehmen liessen. Um den Wirkungsmechanismus des erfindungsgemässen Arzneimittels herauszubekommen, untersuchte man es an Mäusen bei Tests zum Zusammenwirken auf Effekte von Hexenal in einer Menge von 60 mg/kg Körpergewicht des Tieres, von Apomorphin in einer Menge von 2 mg/kg Körpergewicht des Tieres, von Phenamin in einer Menge von 6 mg/kg Körpergewicht des Tieres und von Arekolin in einer Menge von 25 mg/kg Körpergewicht des Tieres, wobei dann die Dauer der latenten Perioden für das Auftreten der Hexenal-, Apomorphin- und Phenamin-Effekte und die Dauer des Hexenalschlafes, der Phenamin- und der Apomorphinstereotypie und der Arekolinhyperkinese eingeschätzt wurde. Die erzielten Angaben sind in Tabelle 5 wiedergegeben. Wie aus der Tabelle ersichtlich ist, wirkt sich die vorherige Einführung des erfindungsgemässen Arzneimittels in einer Menge zwischen 20 und 60 mg/kg Körpergewicht des Tieres lediglich auf die Verlängerung der latenten Periode der Apomorphin- und der Phenaminstereotypie. Die Zeit des Hexenalschlafes und die Dauer sowie die Ausgeprägtheit des Phenamin- und der Apomorphinstereotypie sowie der Arekolinhyperkinese bleiben konstant.

Untersucht wurde der Einfluss des erfindungsgemässen Arzneimittels and Mäusen in einem Test mit Korasol in einer Menge von 150 mg/kg Körpergewicht, dessen Einführung in der genannten Menge einen 100%igen Tod der Tiere nach 2 bis 4 Minuten bewirkte. Die hierbei erhaltenen Daten sind in Tabelle 5 zusammengestellt. Diese Daten lehren, dass die intragastrale Einführung des erfindungsgemässen Arzneimittels 20 min vor der Einführung von Korazol mit einer Verlängerung der latenten Periode von Korasolkrämpfen und mit einer gewissen Unterdrückung der tonischen Extensorkomponente des Krampfanfalls einghergeht, allerdings richten sich die fesgestallten Effekte nach der Menge des erfundungsgemässen Arzneimittels.

Es wurde fernerhin eine vergleichende Untersuchung der Einflussnahme des erfindungsgemässen Arzneimittels und des Phenibuts an Ratten bei Testen zum Zusammenwirken mit Hexenal und Korasol vorgenommen. Die Untersuchungsergebnisse sind in Tabelle 6 wiedergegeben.

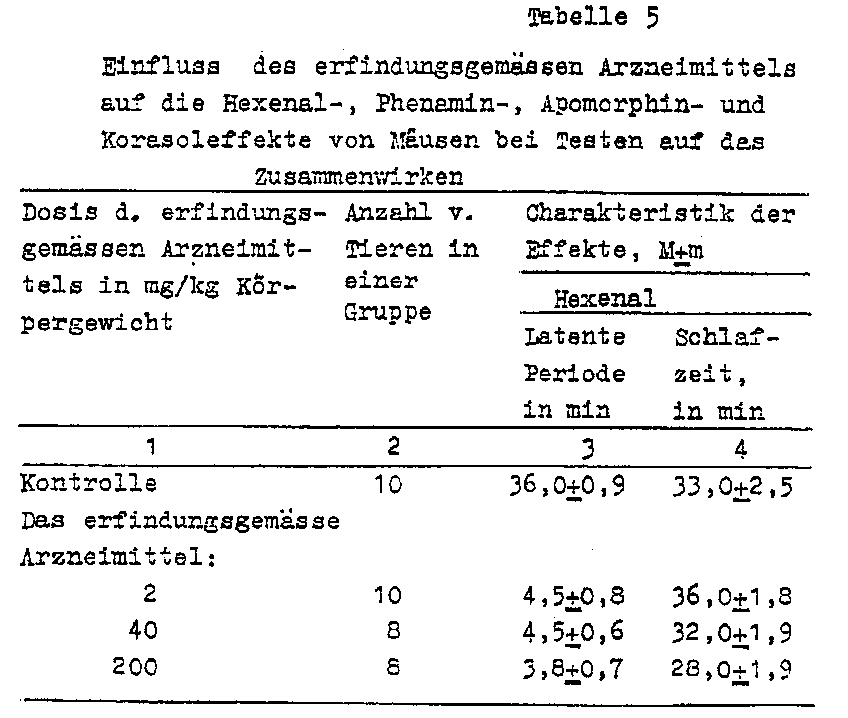

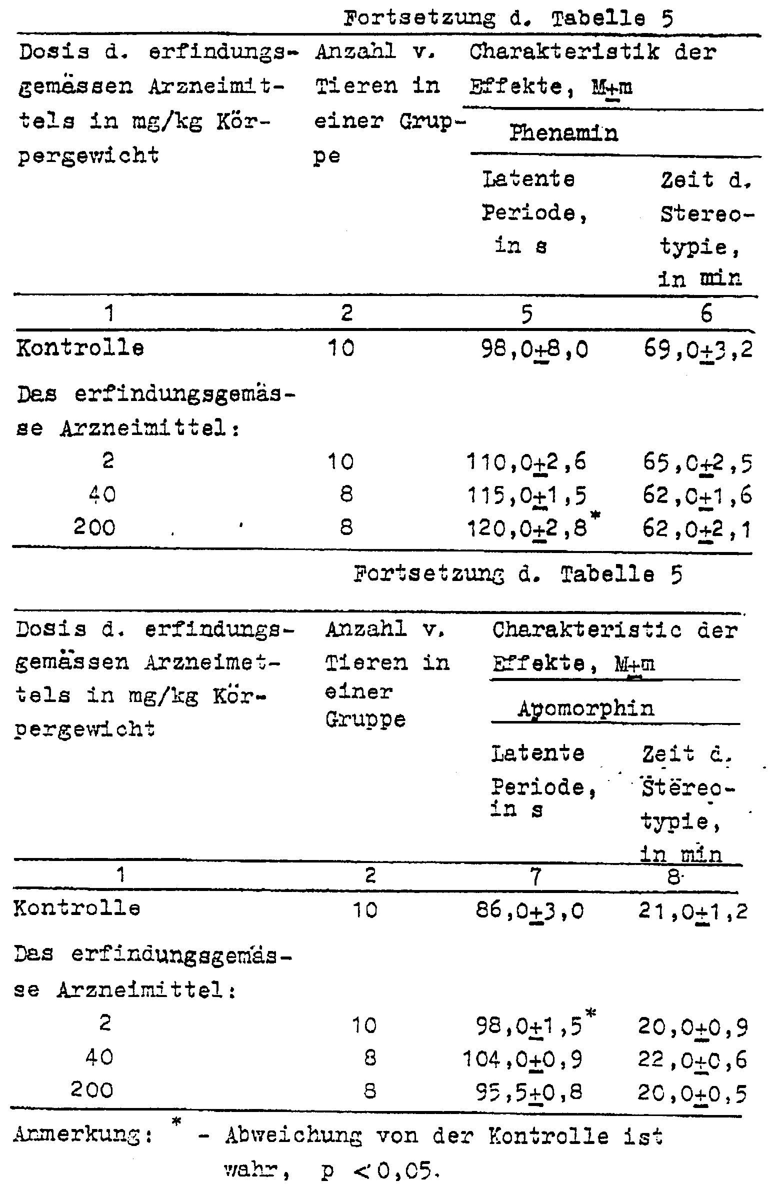

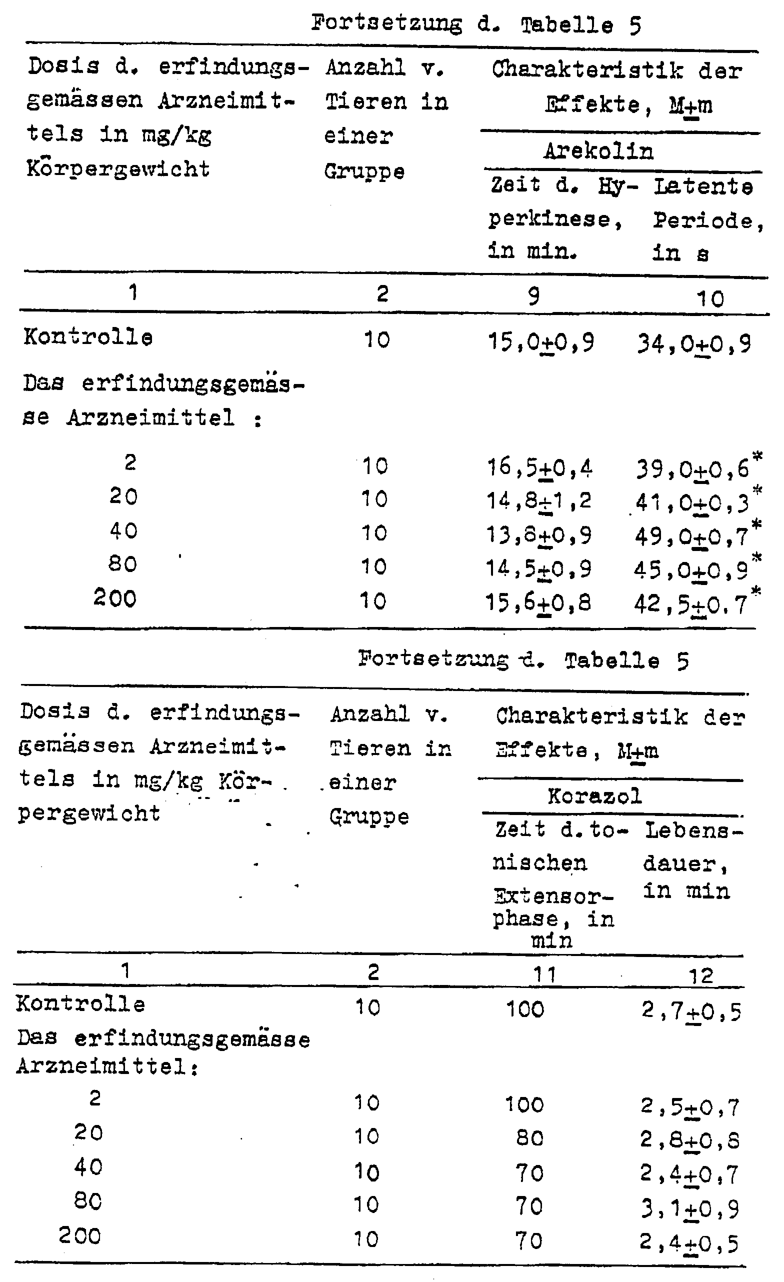

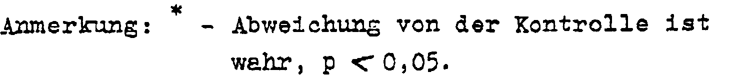

**Tabelle 6**

| Einfluss des erfindungsgemässen Arzneimittels und des Phenibuts bei Testen zum Zusammenwirken auf die Hexenal- und Korasol-Effekte bei Ratten | | |
|---|---|---|
| Mengen in mg/kg Körpergewicht d. Tieres | Hexenal | |
| | Schlafzeit in min, M±m | |
| | Erfindungsgemässes Arzneimittel | Phenibut |
| Kontrolle | 33,0±2,6 | 40,7±4,1 |
| 2 | 36,0±1,8 | - |
| 40 | 32,0±1,9 | - |
| 100 | - | 110,0±8,3 |
| 200 | 28,0±1,9 | 130,0±8,5 |
| Aussagekraft d.Teste, p | > 0,1 | < 0,05 |

| | Korasol | |
|---|---|---|
| | Latente Periode für das Auftreten von Krämpfen in min, M±m | |
| Kontrolle | 34,0±0,9 | 52,0±3,9 |
| 20 | 41,0±0,3 | - |
| 80 | 45,0±0,9 | - |
| 100 | - | 53,0±5,4 |
| 200 | 42,5±0,7 | 50,0±2,8 |
| Aussagekraft d. Teste, p | < 0,05 | - |

Die Angaben der Tabelle weisen darauf hin, dass sich die Effekte des erfindungsgemässen Arzneimittels grundsätzlich von denen das Phenibuts unterschieden, dessen Wirkung der der Tranquillizers aus der Benzdiazepin-Reihe gleicht.

Infolge dessen, dass bei den Testen zum Zusammenwirken mit Korasol eine schwache antikonvulsive Wirkung des erfindungsgemässen Arzneimittels herausgefunden wurde, untersuchte man dessen eventuelle antikonvulsive Aktivität an Mäusen bei Testen mit einer innerlichen Titration von Korasol und Strychnin sowie bei Anwendung des Elektroschocks an Modellen, die es gestatten, den Mechanismus für die Unterrückung der tonischen Extensorkomponente eines Konvulsionsanfalls genauer zu ermitteln. Die dabei erhaltenen Angaben sind in Tabelle 7 zusammengestellt. Die Angaben der Tabelle lassen erkennen, dass das erfindungsgemässe Arzneimittel nahezu keinen Einfluss auf die Strychnin-Effekte ausübt, keine Aktivität gegenüber den durch einen Elektroschok bewirkten Krämpfen aufweist, dennoch die Schwellendosis von Korasol etwas steigert, bei dar ein Anfall losgeht und eine tonische Extensorphase und der Tod von Tieren feststellbar sind. Derartige Angeben sprechen für einen selektiven Antagonismus des erfindungsgemässen Arzneimittels in bezug auf Korasol und nicht für dessen direkten antikonvulsiven Effekt.

Um die Wirkungsmechanismen des erfindungsgemässen Arzneimittels näher zu ermitteln, wurde seine Aktivität in Versuchen an isolierten Objekten mit glatten Muskeln (Samenbläschen der Ratte und Krummdarm des Meerschweinchens) studiert. Mit Hilfe eines Gerätes Mechanotrop und von Mechanotrop-Gebern erfasste man die Kontraktionsaktivität der genannten Objekte in Gegenwart des erfindungsgemässen Arzneimittels, des Acetylcholins und des Adrenalins. Die Untersuchungsergebnisse sind der Tabelle 8 entnehmbar.

**Tabelle 8**

| Einfluss des erfindungsgemässen Arzneimittels auf die Kontraktionsantworten der glattmuskulösen Organe einer Ratte und eines Meerschweinchens | | | | |
|---|---|---|---|---|
| Konzentration d. erfindungsgemässen Arzneimittels in M | Kontraktionsamplitude in mm (M±m) und in % bezogen auf die Kontrolle | | | |
| | Agonisten | | | |
| | Adrenalin (Samenbläschen d. Ratte) | | Azetylcholin (Krummdarm d.Meerschweinchens) | |
| Kontrolle | 69,0±2,8 | 100% | 107,0±2,5 | 100% |

| Erfindungsgemässes Arzneimittel: | | | | |
|---|---|---|---|---|
| 1·10⁻⁶ | 65,0±1,2 | 94% | 116,0±2,4 | 109% |
| 1·10⁻⁵ | 57,0±0,8* | 83% | 105,0±1,8 | 99% |
| 1·10⁻⁴ | 45,0±0,6* | 65% | 97,0±1,5 | 89% |

| | | | | |
|---|---|---|---|---|
| * die Abweichung von der Kontrolle ist wahr, p < 0,05. | | | | |

Die Angaben der Tabelle deuten an, dass durch das erfindungsgemässe Arzneimittel die Kontraktionsantworten auf das Adrenalin der Samenbläschen unterdrückt werden, ohne dass sich ein Antagonismus mit dem Acetylcholin zeigt. Die schwache und mässige adrenalinsperrende Wirkung des erfindungsgemässen Arzneimittels in einer Konzentration von 1·10⁻⁵ M und 1·10⁻⁴ M zeugt davon, dass es in der Lage ist, mit adrenergischen Rezeptoren, insbesondere Alpha₁-Adrenorezeptoren, zusammenzuwirken. Ausgehend von der Analogie mit anderen bekannten pharmakologischen Präparaten kann angenommen werden, dass eine solche Wechselwirkung mit den Alpha₁-Adrenorezeptoren des erfindungsgemässe Arzneimittel auch mit den Adrenorezeptoren des Gehirnes aufweist, wodurch eben seine Antistressaktivität bei einem akuten und dauernden Stress, der mit einem Adrenalinanstieg in den Geweben einhergeht, bedingt ist.

Untersucht worden ist die Einflussnahme des erfindungsgemässen Arzneimittels auf die spontane bioelektrische Aktivität des Gehirnes von Ratten, welche bei den Tieren mit eingepflanzten Elektroden bei freiem Verhalten erfasst wurde. Die diesbezüglichen Ergebnisse sind in Tabelle 9 wiedergegeben. Diese Ergebnisse der Tabelle sprechen dafür, dass das erfindungsgemässe Arzneimittel in einer Menge von 40 mg/kg Körpergewicht des Tieres, nachdem es intraabdominal eingeführt wurde, eine synchronisierte Wirkung am Enzephalogramm verfolgen liess, was sich durch eine Vergrösserung der Amplitude und des Indexes für den Langsamrhythmus des EEG ausdrückte. Jedoch die Wirkung des erfindungsgemässen Arzneimittels unterscheidet sich von der der bekannten Arzneimittel, welche einen synchronisierten Effekt auslösen und welche durch den Effekt in Form von wiederholten Episoden mit einer Häufigkeit von 1 bis 2 Male in der Stunde bei einem 4 Stunden langen Versuch die Amplitude und den Index des Langsamrhythmus beeinflussen.

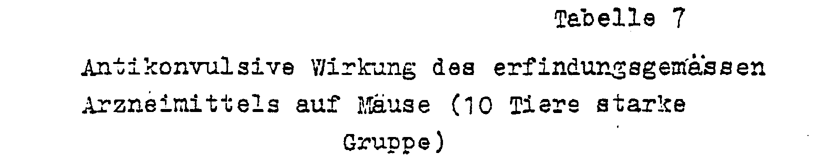

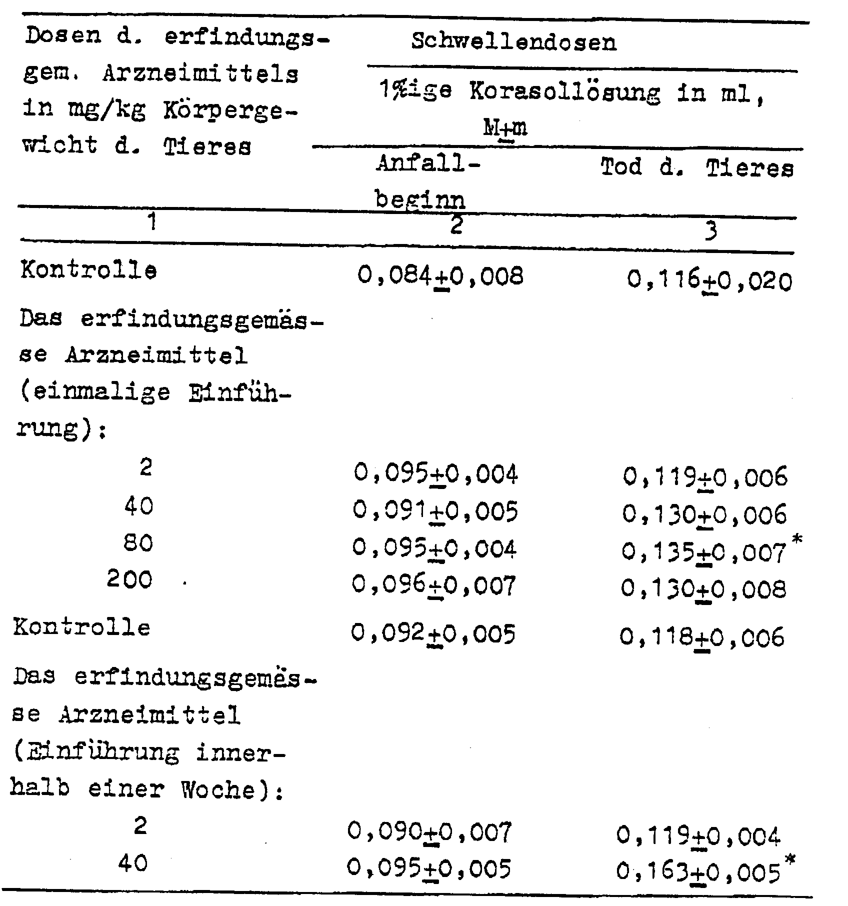

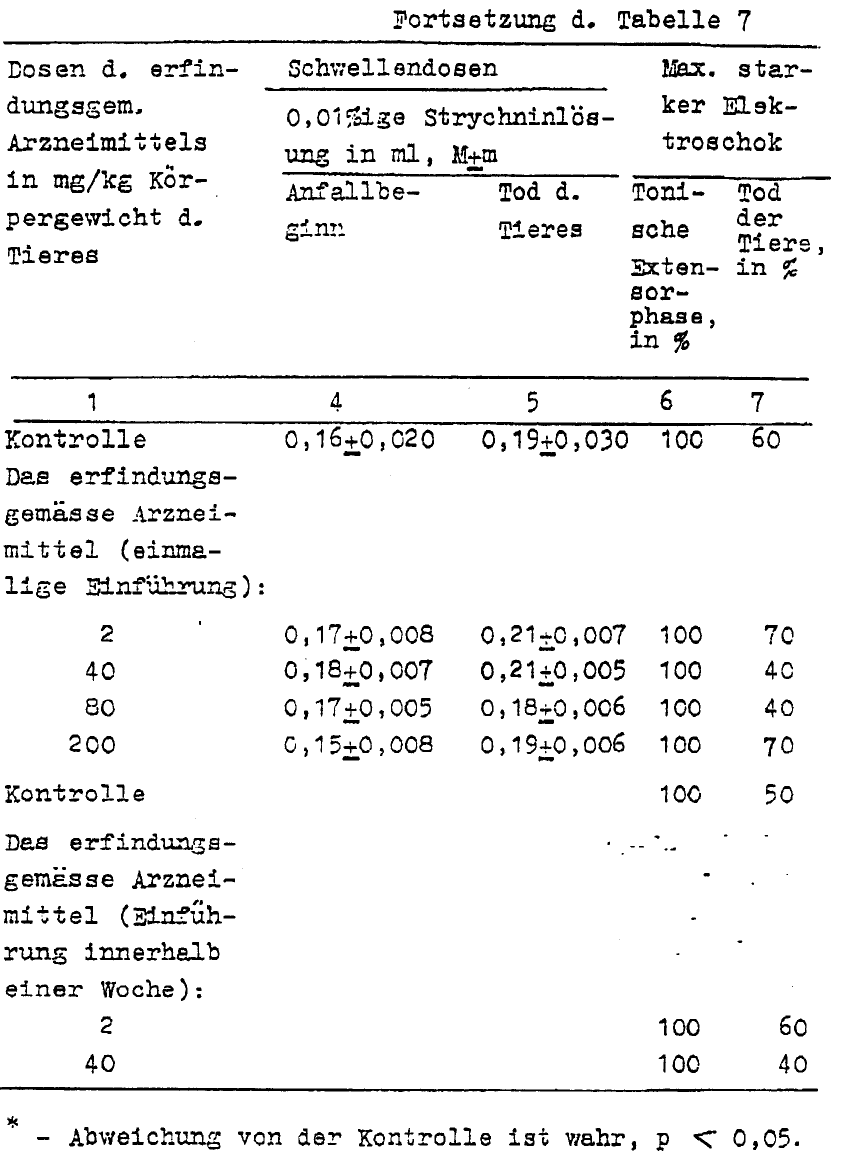

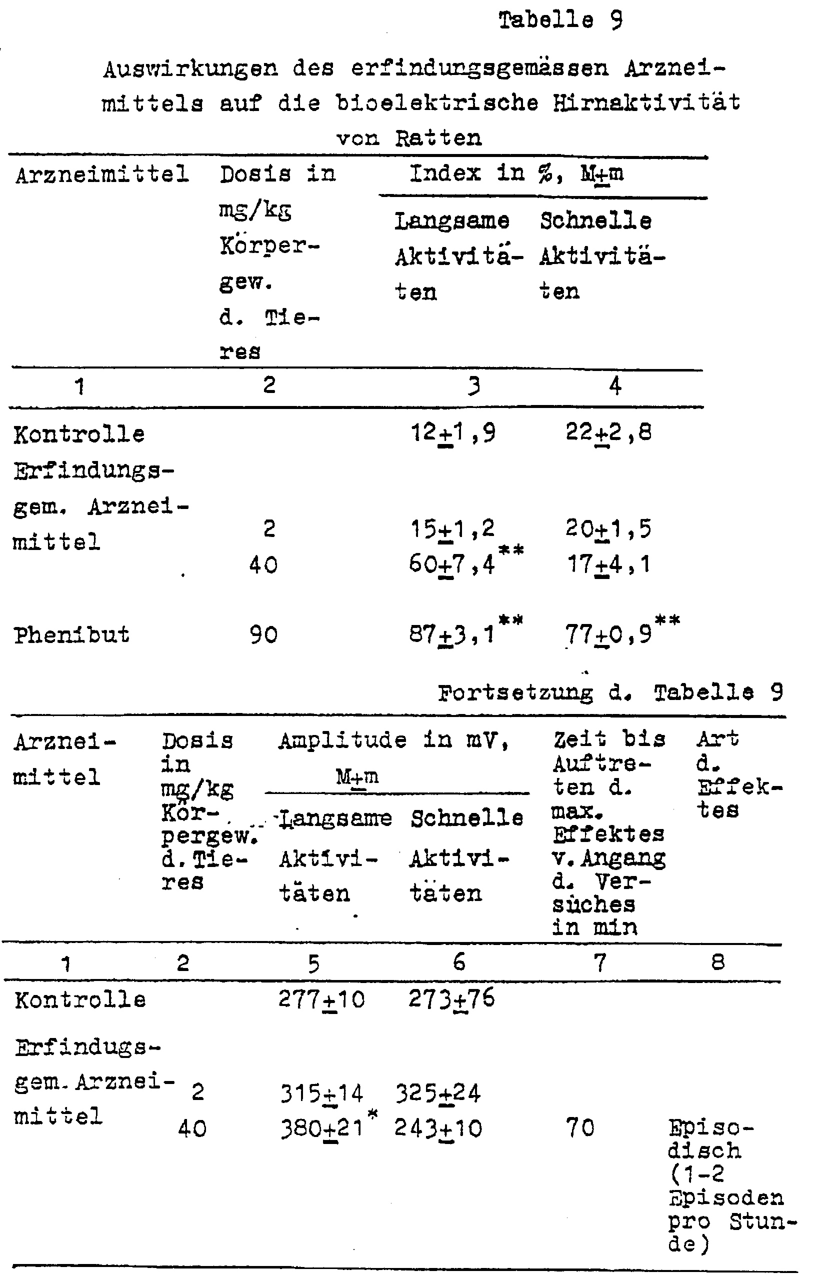

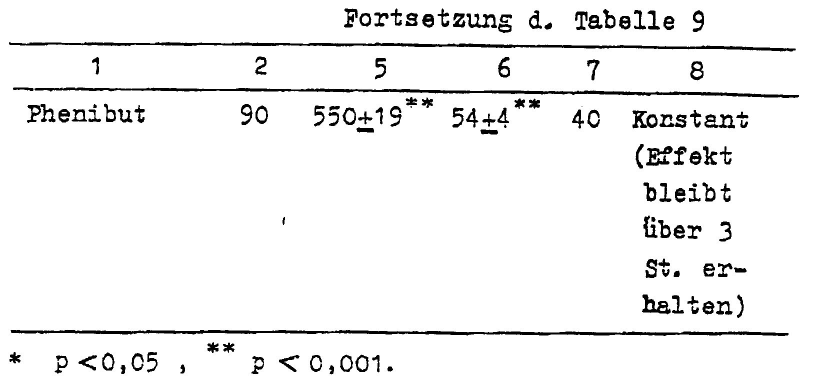

Die durchgeführten versuchsweisen Untersuchungen haben somit ergeben, dass das erfindungsgemässe Arzneimittel eine Antistress- und eine Stressschutzwirkung aufweist, neurotrope Eigenschaften hat und dank seiner beruhigenden und für das EEG synchronisierende Wirkung den für die Tranquillizers und die GAMK-Derivate Phenibut , typischen Eigenschaften beraubt ist, welche bei Vergrösserung der Dosis die Bewegungsaktivität gewaltig bremsen, die Wirkung von Hexenal potenzieren, die Zeit des Hexenal-Schlafes vergrössern und zugleich die tonische Extensorkomponente der Korasol- und Strychninkrämpfe sowie des Elektroschocks abschwächen.

Das erfindungsgemässe Arzneimittel ist bei einer Dosis von 1 bis 2 mg/kg Körpergewicht am wirksamsten, und bei deren Anstieg bleibt die Grösse der Änderungen, bedingt durch die wirksame Dosis, erhalten, was ebenfalls einen grundsätzlichen Unterschied des erfindungsgemässen Arzneimittels von Benzdiazepinen und GAMK-Derivaten darstellt.

Die LD₅₀ des erfindungsgemässen Arzneimittels wurde an Mäusen (eine Gruppe aus 50 Tieren mit einer Masse von je 25 bis 30 g) und an Ratten (eine Gruppe aus 30 Tieren mit einer Messe von je 200 bis 250 g) festgestellt. Man hat gefunden, dass bei intragastraler Einführung des erfindungsgemässen Arzneimittels die LD₅₀ mehr als 3000 mg/kg Körpergewicht des Tieres ausmacht, so dass das erfindungsgemässe Arzneimittel nicht toxisch ist.

Eine pathomorphologische Untersuchung der inneren Organe von Tieren nach mehrfacher intragastraler Einführung des erfindungsgemässen Arzneimittels innerhalb von 60 Tagen in einer Menge von je 200 mg/kg Körpergewicht des Tieres ergab keine morphologischen und pathologischen Änderungen.

Untersucht wurde der Einfluss des erfindungsgemässen Arzneimittels auf die geistige Arbeitsfähigkeit, seine Antistress- und Stressschutzwirkung an 20 gesunden Menschen, 33 Alkoholikern und an 154 Kindern und Schuljungen. Die Kontrollgruppen aus den Erwachsenen, Kindern und Halbwüchsigen bekamen ein Placebo und nicht das erfindungsgemässe Arzneimittel verabreicht. Das erfindungsgemässe Arzneimittel wurde einmal in einer Menge von 2 mg/kg Körpergewicht als Unterzungenmittel oder bei einer Kur innerhalb von 14 bis 30 Tage in einer Menge von 2 mg/kg Körpergewicht 2 bis 3 mal pro Tag eingenommen. Man studierte die Einflussnahme des erfindungsgemässen Arzneimittels unter Verwendung besonderer Teste (Tabellen von Krepelin, Schulte u.a.), die es ermöglichen, die Geschwindigkeit von Rechenoperationen, die Aufmerksamkeit, das kurzfristige Gedächtnis, den Zustand des Zentralnervensystems, die sensomotorischen Reaktionen und den Zustand des Herz- und Gefässsystems zu beurteilen.

Um den Einfluss des erfindungsgemässen Arzneimittels auf die Geschwindigkeit der Zähl- und Rechenoperationen zu bestimmen, bediente man sich einer Tabelle mit 184 Paaren von Primzahlen, die mit Rücksicht auf die Zeit für die Erfüllung der Aufgabe paarweise addiert wurden. Dieser Test ermöglicht es, die Schnelligkeit der Zähl- und Rechenoperationen in einer Zeiteinheit (einer Minute), die Zeit für die Erfüllung der Aufgabe (die Zeit für das Addieren von 184 Paaren von Zahlen), die Anzahl von Fehlern, die Schnelligkeit der Umschaltung der Aufmerksamkeit und den Ermüdungsgrad zu bewerten. Das Studium des Einflusses der einmaligen Einnahme des erfindungsgemässen Arzneimittels auf die Schnelligkeit der Zähl- und Rechenoperationen hat ergeben, dass 30 min nach Einnahme des erfindungsgemässen Arzneimittel die Kinder und Halbwüchsigen in 79,5% der Fälle die Zeit für das Addition von Zählen (um 1,5 bis 2 min) weniger aufwandten und die Anzahl von Fehlern beim Zählen doppelt so wenig war. In der Kontrollkindergruppe beobachtete man 30 min nach Einnahme von Placebo keine wesentlichen Änderungen. Untersucht wurde der Einfluss des erfindungsgemässen Arzneimittels auf die Geschwindigkeit der Zähl- und Rechenoperationen, die durch praktisch gesunde Leute und am Alkoholismums kranke Leute ausgeführt wurden. Die Ergebnisse der Untersuchung sind in Tabellen 10 und 11 wiedergegeben.

**Tabelle 10**

| Einfluss des erfindungsgemässen Arzneimittels auf die Geschwindigkeit der Zähl- und Rechenoperationen von praktisch gesunden Leuten | | | | | |
|---|---|---|---|---|---|
| Stufen d. Untersuchung | Zahl d. Probanden | Additionsgeschw.in Paar/min, M±m | Anzahl v. Fehlern | Zahl v. Probanden,die Fehler machten in % | Aussagekraft der Teste, p |
| 1 | 2 | 3 | 4 | 5 | 6 |
| Vor Einnahme von Placebo | 20 | 48,0±1,1 | 2 | 85 | |
| 30 min nach d. Einnahme von Placebo | 20 | 47,0±1,7 | 1,9 | 80 | - |
| Vor Einnahme d. erfindungsgem. Arzneimit. | 20 | 27,0±1,8 | 2 | 80 | < 0,05 |
| 30 min nach d. Einnahme d. erfindungsgem. Arzneimittels | 20 | 52,0±1,9 | 1 | 40 | |

**Tabelle 11**

| Einfluss des erfindungsgemässen Arzneimittels auf die Geschwindigkeit der Durchführung von Zähl und Rechenoperationen durch am Alkoholismus kranke Leute | | | | |
|---|---|---|---|---|
| Gruppen v. Kranken | Zahl v. Aprobanden | Stufe d. Untersuchung | Additionsgeschwindigkeit, in Paaren/min, M±m | Aussagekraft d. Versuche, p |
| Alkoholabstinenzsyndrom (2 Tage) | 10 | Vor Einnahme d. erfindungsgem. Arzneimit. | 28,0±1,6 | |
| | | Nach Einnahme d. erfindungsgem. Arzneimittels | 32,0±1,6 | < 0,05 |
| Alkoholismus | 35 | Vor Einnahme d. erfindungsgem. Arzneimit. | 47,0±1,2 | < 0,05 |
| | | Nach Einnahme d. erfindungsgem. Arzneimit. | 51,0±1,8 | |

Untersucht wurde der Einfluss des erfindungsgemässen Arzneimittels auf die Aufmerksamkeit, das kurzzeitige Gedächtnis und die sensomotorischen Reaktionen von Schulkindern. Die Untersuchung auf die Aufmerksamkeit erfolge unter Verwendung des Schult-Testes. Bei diesem Test wird dem Probanden eine Tafel mit 49 Karos angeboten, unter denen durcheinander 24 rote und 25 schwarze Zahlen angeordnet sind. Die Zahlen sollen in einer strikten Reihenfolge wie z. B. die roten Zahlen in steigender und die schwarzen in abnehmender Reihenfolge gefunden werden. Hierbei wird die Zeit für die Erfüllung der gesamten Aufgabe für die Suche nach jeder Zahl gemessen und die Anzahl Fehlern erfasst. Dank diesem Test kenn man die Aufmerksamkeit beurteilen und Leute mit erhöhter Emotionalität ausfindig machen.

Um den Zustand dem Kurzzeitigen Gedächtnisses zu studieren, bediente man sich eines Testes, der auf der Wiedergabe von 10 zweistelligen Zahlen, welche 20 s lang auf einem Bildschirm angezeigt wurden, beruht. Bei dem weiteren Testen wurde eine neue Folge von Zahlen angezeigt. Die sensomotorischen Reaktionen bewertete man mit Hilfe einer visuellmotorischen Reaktion (Differentiation von Sehsignalen aus 20 Prämissen).

Die Versuchs- und die Kontrollgruppe machten die Teste zweimal voll durch: der erste Test galt als Bezugstest, wonach die Versuchsgruppe der Kinder das erfindungsgemässe Arzneimittel einmal in einer Menge von 2 mg/kg Körpergewicht und die Kontrollgruppe ein Placebo verabreicht bekamen, und dann wurde der zweite Test ausgeführt.

Die Untersuchungen haben ergeben, dass alle Schüler die erste Serie von Testen (Bezugstesten) voll durchmacht hatten, doch die meisten von ihnen verwiesen auf eine Ermüdung, die bei 10,4% ausgeprägt, bei 18,7% mässig und bei 62,5% leicht war, dabei sagten 87,5% der Probanden aus, dase die grössten Schwierigkeiten für sie das Behalten zweistelliger Zahlen wegen einer grossem Erregung und keiner Möglichkeit für die Konzentration sowie die Suche nach den Zahlen mit der Umschaltung (Schulte-Test) machten. Der letzte Test löste eine ausgeprägte neuropsychische Spannung der Probanden aus, die mit einer Gesichtserrötung, einem Händezittern und einem höheren Schwitzen einherging. Beim nochmaligen Testen der Versuchsgruppe stellte man einen positiven Einfluss des erfindungsgemässen Arzneimittels auf den emotionellen Zustand, die geistige Arbeitsfähigkeit, das Gedächtnis und die Aufmerksamkeit fest. Nach der Einnahme des erfindungsgemässen Arzneimittels beobachtete man in 87,2% der Fälle eine subjektive und objektive Verbesserung des Gesamtbefindens wie das Verschwinden der Müdigkeit, das Eintreten einer höheren Konzentration und eines ruhigeren Zustands bei der Ausführung der Teste. Die Ergebnisse für das zweite (hauptsächliche) Testen sind in Tabelle 12 wiedergegeben.

Die Angaben aus der Tabelle 12 weisen darauf hin, dass sich die Kapazität des kurzzeitigen Gedächtnisses im Durchschnitt um 25% in 58,9 der Fälle verbesserte und sich bei 25,7% der Probanden nicht verschlechterte und auf der ursprünglichen Ebene erhalten bleib. Bei 56,4% der Schüler stellte man unter Einfluss des erfindungsgemässen Arzneimittels eine Verkürzung der latenten Periode der visuellmotorischen Reaktion und eine niedrige Zahl von Differenzierungsfehlern fest. Die Einnahme des erfindungsgemässen Arzneimittels beeinflusste im wesentlichen auch die Aufmerksamkeit, indem die Zeit für das Umschalten der Aufmerksamkeit und die Zahl der gemachten Fehler reduziert wurden.

Bei den Kindern, denen ein Placebo verabreicht wurde, beobachtete man in 65% der Fälle bei nochmaliger Untersuchung eine Erhöhung der Müdigkeit, eine Verschlechterung der Arbeitsfähigkeit (Verschlechterung des Gedächtnisses um 20% und der Parameter der visuellmotorischen Reaktion, eine längere Zeit für das Umschalten der Aufmerksamkeit).

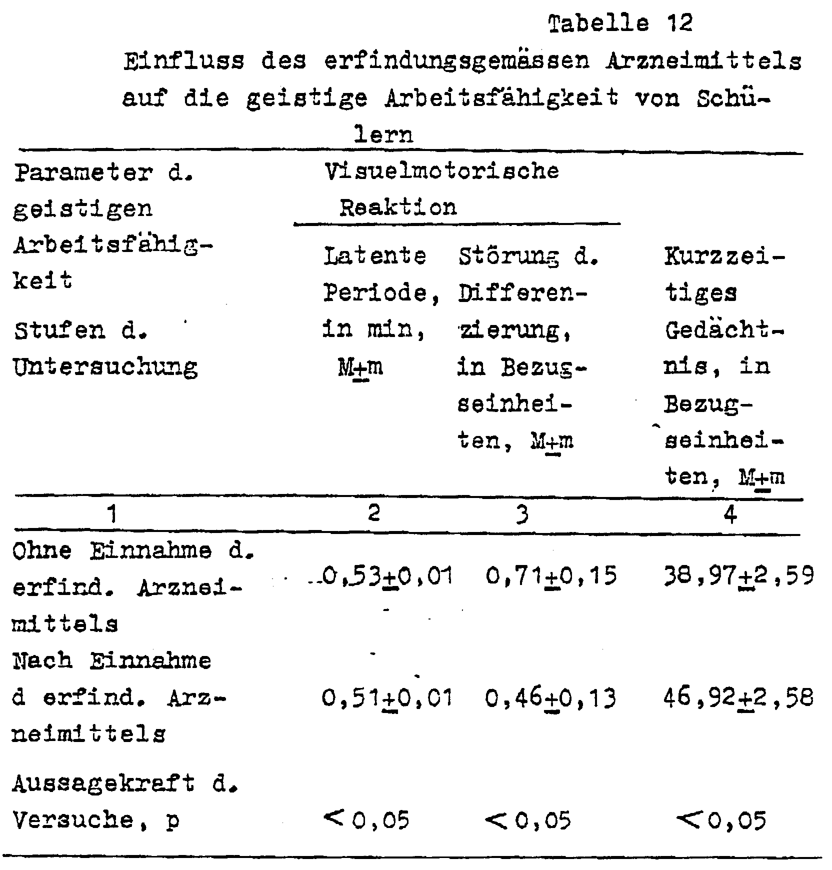

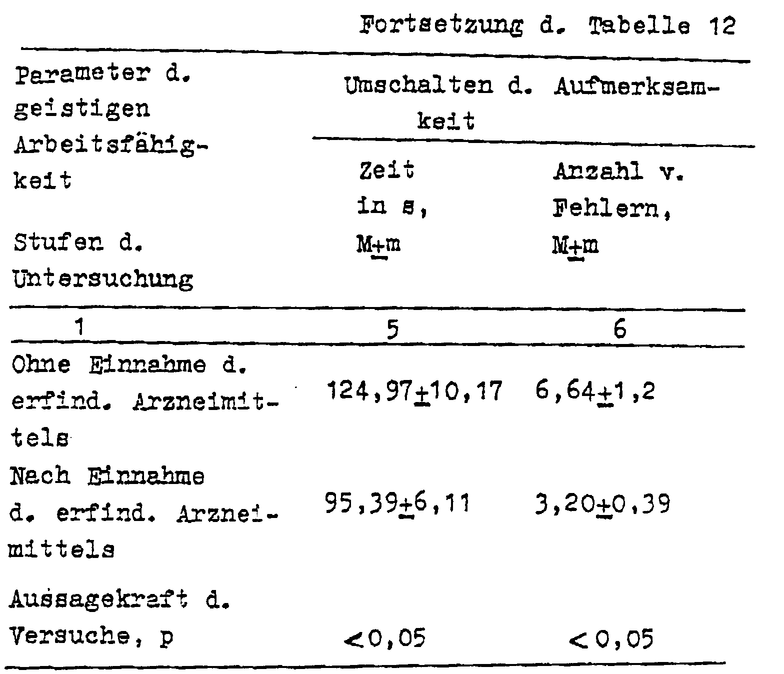

Neben der Bewertung der Parameter, die sich auf die geistige Arbeitsfähigkeit beziehen, ermittelts man bei den Schülern in der Testierperiode und beim Ablegen von Prüfungen die Parameter des Pulses und des Arteriendruckes, die im wesentlicher den Grad der neuropsychischen Spannung wiedergeben Die erhaltenen Daten sind in Tabelle 13 zusammengestellt.

Die Angaben der Tabelle 13 lassen erkennen, dass unter Einfluss des erfindungsgemässen Arzneimittels der Puls und der Arteriendruck keine Änderungen erfahren haben, was unter Bedinungen einer erhöhten neuropsychischen Spannung sonst der Fall ist. Nachdem die Schüler das erfindungsgemässe Arzneimittel 30 min vor dem Examen eingenommen hatten, stellten 95% der Schüler das Verschwinden der Angst und des innerlichen Unbehagens fest. Beobachtungen des Prüfers wiesen nach, dass das Benehmen der Kinder bei der Vorbereitung und dem Ablegen der Prüfungen ruhiger war, dass die Kinder rascher die richtige Antwort fanden und dass ihre Zensuren um 0,5 bis 1 Punkt, insbesondere in der Mathematik, besser lagen als Jahresdurchschnitt (80% machten die Zensuren 2 und 1, in der Kontrollgruppe nur noch 60%, aus). Man untersuchte elektrokardiographisch den Einfluss des erfindungsgemässen Arzneimittels auf die Parameter des Herzrhythmus von Schülern bei dessen einmaliger Einnahme. Die erzielten Angaben sind in Tabelle 14 wiedergegeben. Wie dar Tabelle 14 zu entnehmen ist, übt das erfindungsgemässe Arzneimittel einen günstigen Einfluss auf den Zustand des Organismus der Probanden bei einer neuropsychischen Spannung aus.

Untersucht ist die Einflussnahme des erfindungsgemässen Arzneimittels bei dessen dauernder Einnahme auf die geistige Arbeitsfähigkeit der Schüler der unteren Klassen, die lehrbehindert sind und eine Korrektionsklasse besuchen. Die Kontrolle über die Effizienz des erfindungsgemässen Arzneimittels erfolgte unter Benutzung von Korrektionstesten. Man untersuchte die Dynamik der geistigen Arbeitsfähigkeit der Probanden in zwei Stufen. Innerhalb der ersten Arbeitswoche wurde jeden Tag des Anfangsniveau der Arbeitsfähigkeit ermittelt, im Laufe der zweiten Arbeitswoche studierte man bei denselben Kindern dieselben Parameter der Arbeitsfähigkeit unter experimentellen Bedinungen vor Hintergrund der Einnahme des erfindungsgemässen Arzneimittels durch die Kinder. Die Ergebnisse der Untersuchung sind in Tabelle 15 Wiedergegeben. Die Angaben der Tabelle 15 zeugen davon, dass bei den Schülern vor Hintergrund der Einnahme des erfindungsgemässen Arzneimittels ein relativ wesentlicher Rückgang dar Fehlerzahl pro 500 Zeichen vor und nach dem Unterricht sowie ein höherer Prozentsatz von fehlerfreien Aufgaben feststellbar waren. Diese Resultate sprechen für einen positiven Effekt des erfindungsgemässen Arzneimittels auf die Funktion Aufmerksamkeit und Stimulation des funktionellen Zustandes des Zentralnervensystems.

Man untersuchte den Einfluss des erfindungsgemässen Arzneimittels bei dessen einmaliger und dauernder Einnahme (14 Tage lang) auf den psychoemotionellen Zustand und auf die Parameter des Elektroenzephalogramms von Halbwüchsigen mit Deviationsformen des Verhaltens. Klinische und psychologische Studien ergaben, dass nach Einnahme des erfindungsgemässen Arzneimittels die Ermüdung der Probanden, deren Reizbarkeit und die innere Unruhe im Wesentlichen zurückgingen. Die Ergebnisse der Auswertung des EEG sind in Tabellen 16 und 17 wiedergegeben. Aus diesen Tabellen ist es ersichtlich, dass das EEG der Probanden eine positive Entwicklung der Parameter sowohl nach einmaliger als auch nach dauernder Einnahme des erfindungsgemässen Arzneimittels aufwies.

**Tabelle 13**

| Einfluss des erfindungsgemässen Arzneimittels auf die Parameter der Entwicklung des Pulses und des Arteriendruckes von Schülern bei geistiger Belestung unter Bedinungen einer erhöhten neuropsychischen Spannung | | | |
|---|---|---|---|
| Stufen der Untersuchung | Pulsfrequanz, in Schlag/min, M±m | Arteriendruck, in mm QS, M±m | |
| | | Systolisch | Diastolisch |
| 1 | 2 | 3 | 4 |
| Laboruntersuchungen: | | | |
| I Bezugsuntersuchungen (v. Einnahme d. erfindungsgem. Arzneimittels): | | | |
| a) vor Testen | 79,5± 1,8 | 110,1± 1,9 | 63,6± 1,5 |
| b) nach Testen | 89,8± 2,1 | 115,6± 2,8 | 71,5± 1,8 |
| Aussagekraft d. Versuche, p | < 0,05 | - | < 0,05 |

| II Hauptsächliche Untersuchungen (n. Einnahme d. erfindunsgem. Arzneimittels): | | | |
|---|---|---|---|
| a) for Testen | 76,8± 1,4 | 110,2± 1,7 | 69,3± 1,3 |
| b) nach Testen | 78,1± 1,4 | 107,7± 1,5 | 69,2± 1,1 |

| Untersuchungen während d. Examina: | | | |
|---|---|---|---|
| I for Examina (Ohne Einnahme d. erfindungsgem. Arzneimittels) | 95,7± 2,1 | 137,7± 3,1 | 70,0± 2,0 |

| II Nach Examena: | | | |
|---|---|---|---|
| a) nach Einnahme d. erfindungsgem. Arzneimittels | 72,8± 2,3 | 112,8± 2,8 | 65,7± 1,5 |
| b) ohne Einnahme d. erfindungsgem. Arzneimittels | 94,0± 2,4 | 140,0± 1,7 | 75,4± 1,8 |
| Aussagekraft d. Versuche, p | < 0,05 | < 0,05 | < 0,05 |

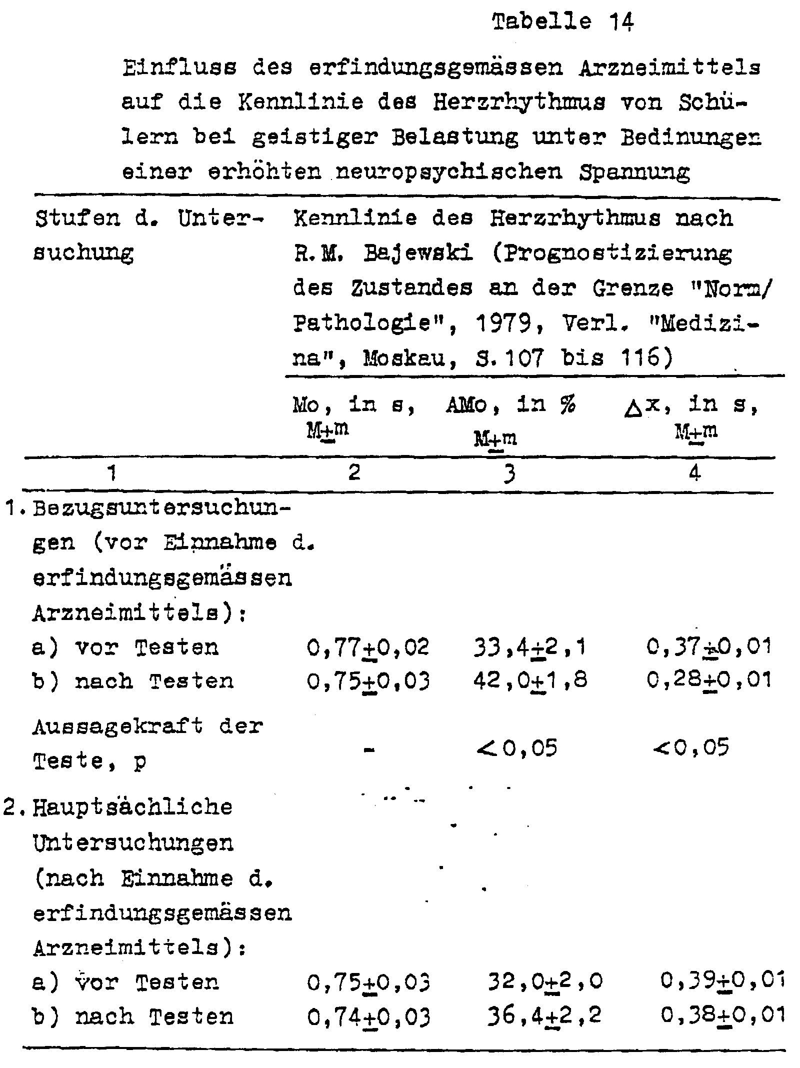

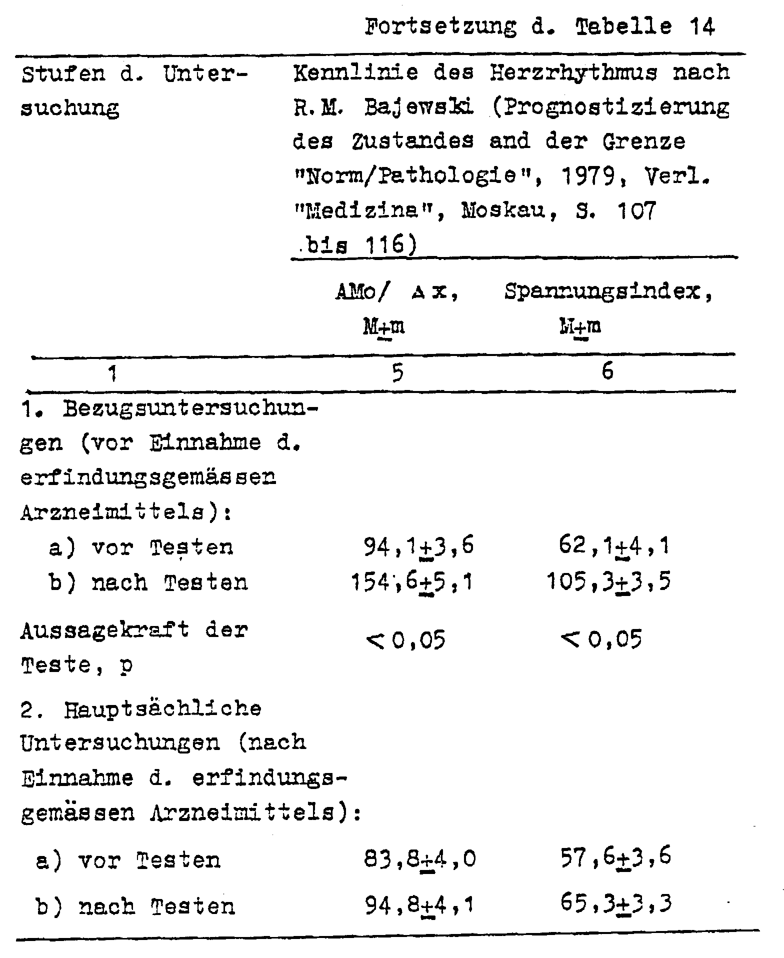

**Tabelle 15**

| Einfluss des erfindungsgemässen Arzneimittels auf die geistige Arbeitsfähigkeit der Schuler von unteren Klassen | | | | |
|---|---|---|---|---|
| Kenndaten d. geistigen Arbeitsfähigkeit | Stufen der Untersuchung | | | |
| | 2. Tag d. Woche | | | |
| | Vor Unterricht | | Nach Unterricht | |
| | Ohne Einnahme d. erfind. Arzneimittels | Nach Einnahme d. erfind. Arzneimittels | Ohne Einnahme d. erfind. Arzneimittels | Nach Einnahme d. erfind. Arzneimittels |
| 1 | 2 | 3 | 4 | 5 |
| Fehlerzahl pro 500 Zeichen, M±m | 11,4±2,6 | 6,5±1,6 | 12,1±1,6 | 7,8±1,8 |
| Aussagekraft d. Teste, p | > 0,05 | | < 0,05 | |
| % der Aufgaben ohne Fehler | 0 | 16,5 | 0 | 25,0 |

| | 4. Tag d. Woche | | | |
|---|---|---|---|---|
| | Vor Unterricht | | Nach Unterricht | |
| | Ohne Einnahme d. erfind. Arzneimittels | Nach Einnahme d. erfind. Arzneimittels | Ohne Einnahme d. erfind. Arzneimittels | Nach Einnahme d. erfind. Arzneimittels |
| 1 | 6 | 7 | 8 | 9 |
| Fehlerzahl pro 500 Zeichen, M±m | 14,7±2,4 | 7,6±1,9 | 14,9±2,4 | 8,4±1,2 |
| Aussagekraft d. Teste, p | < 0,05 | | < 0,05 | |
| % der Aufgaben ohne Fehler | 21,4 | 28,6 | 0 | 14,3 |

Ausgehend von allen studierten Parametern des EEG wurde darüber hinaus gefunden, dass das erfindungsgemässe Arzneimittel durch eine nootrope Aktivität gekennzeichnet ist und dass dessen Wirkung zur Optimierung des funktionellen Zustandes des Zentralnervensystems und zur Normalisierung der Hirnreaktivität beiträgt. Nach der Einnahme des Placebos wurden bei den Probanden keine statisch bedeutsamen Änderungen der EEG-Parameter (Tab. 18) fastgestellt. Die Angaben aus Tabelle 18 weisen nach, dass der "Placebo-Effekt" sowohl nach einmaliger als auch nach vielfacher Einnahme mit ungleichartigen Änderungen der EEG-Parameter einhergeht, was eben nach der Einnahme des erfindungsgemässen Arzneimittels bei der Entwicklung der EEG-Parameter nicht beobachtet wird.

Demgemäss zeigen sich bei dem erfindungsgemässen Arzneimittel eine ausgeprägte Antistress- und Stressschutzwirkung sowie die Eigenschaft, die geistige Arbeitsfähigkeit sowohl unter normalen Redingungen als auch bei einer neuropsychischen Spannung zu verbessern. Am besten ist das erfindungsgemässe Arzneimittel für die Verbesserung der geistigen Arbeitsfähigkeit bei Neurosen, neuroseähnlichen Zuständen, Rasterscheinungen eines Schädel- und Gehirntraumes sowie bei Deviationsformen des Benehmens, den Alkoholismus und asthenovegetativen Störungen geeignet. Es ist ebenfalls als Stressschutzmittel bei psychoemotionellen Spannungen von praktisch gesunden Leuten effizient.

Das erfindungsgemässe Arzneimittel ist voll gefahrlos, zeigt eine milde Wirkung und hat keine Gegenindikationen.

### Gewerbliche Anwendbarkeit

Das erfindungsgemässe Arzneimittel mit einer Antistress-, Stressschutz- und nootropen Wirkung findet in der Praxis für Kinder und Erwachsene bei gestörter Aufmerksamkeit und gestörtem Gedächtnis, verschlechterter geistiger Tätigkeit, bei Neurosen, neuroseähnlichen Zuständen, Resterscheinungen eines Schadel- und Gehirntraumas, einer verzögerten psychischen Entwicklung, Deviationsformen des Benehmens, den Alkoholismus und neuropsychischen Spannungen Anwendund.

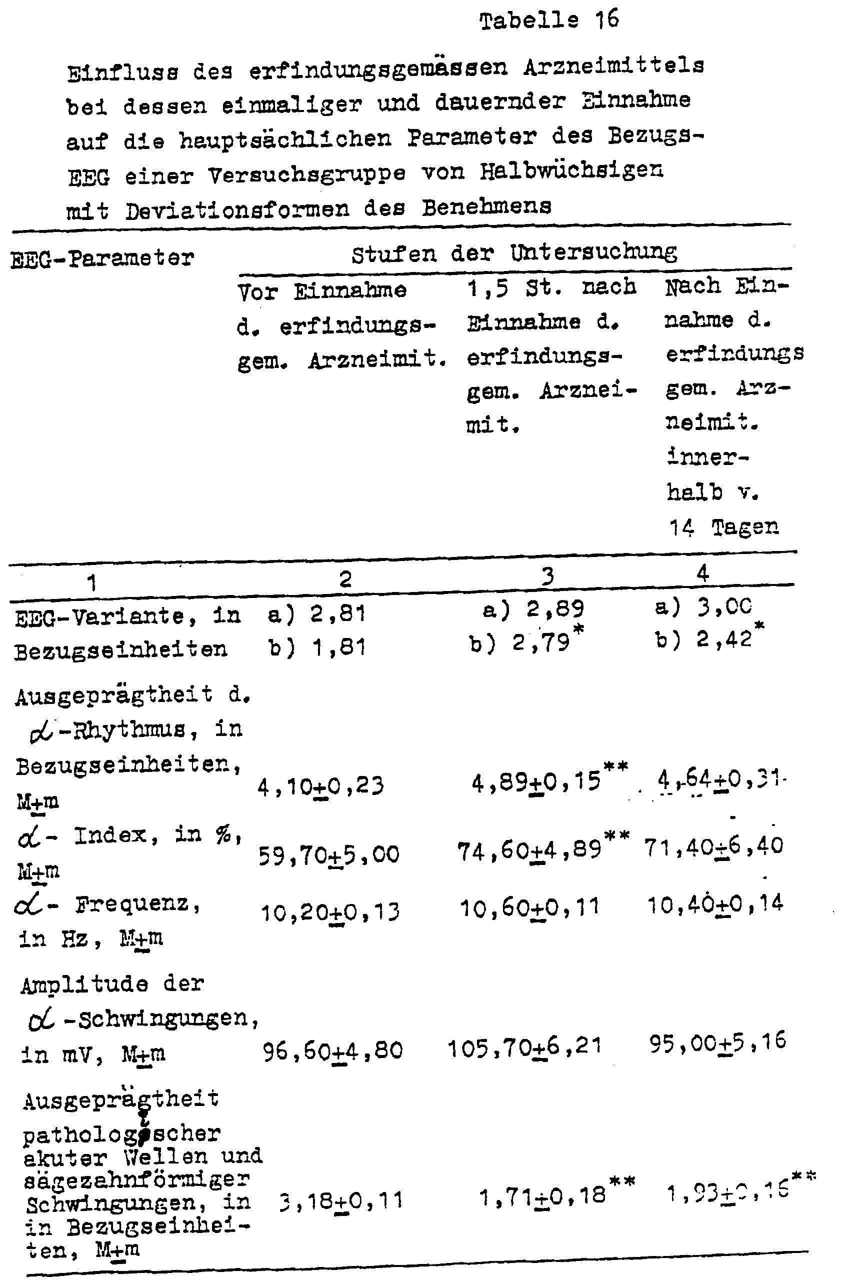

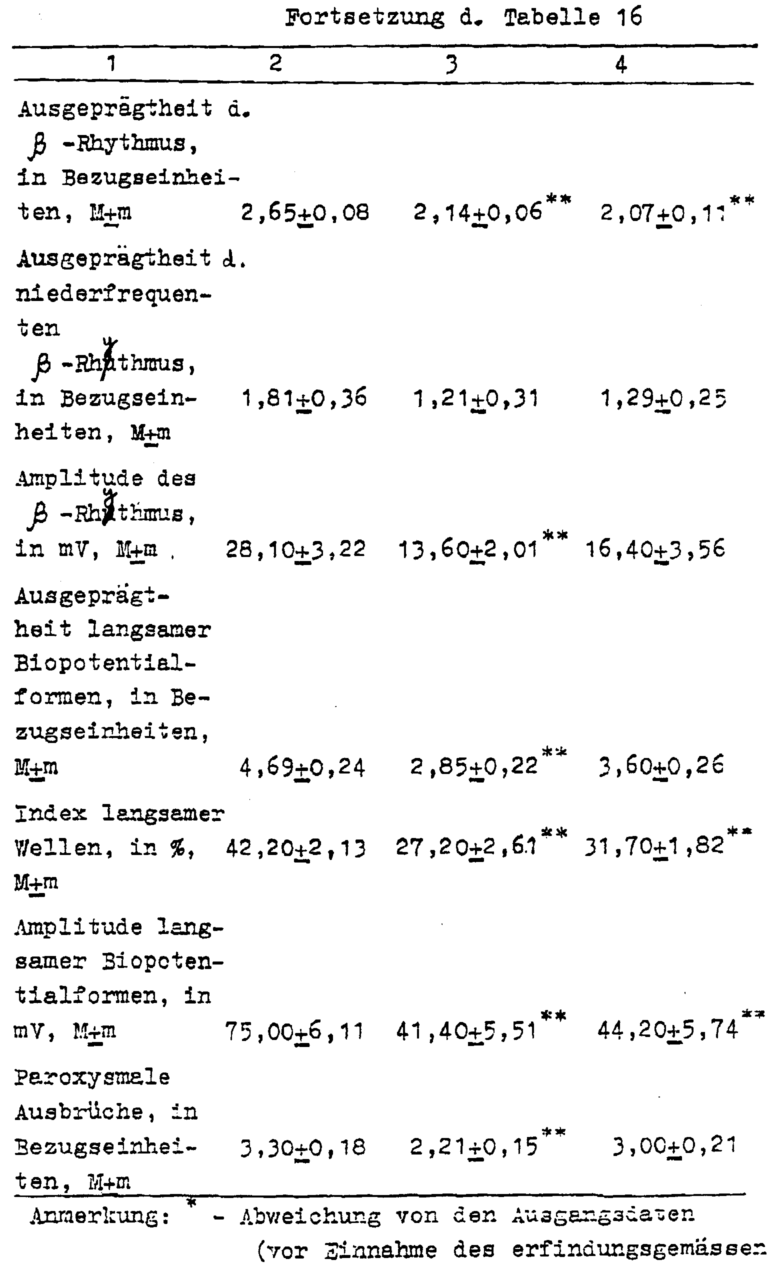

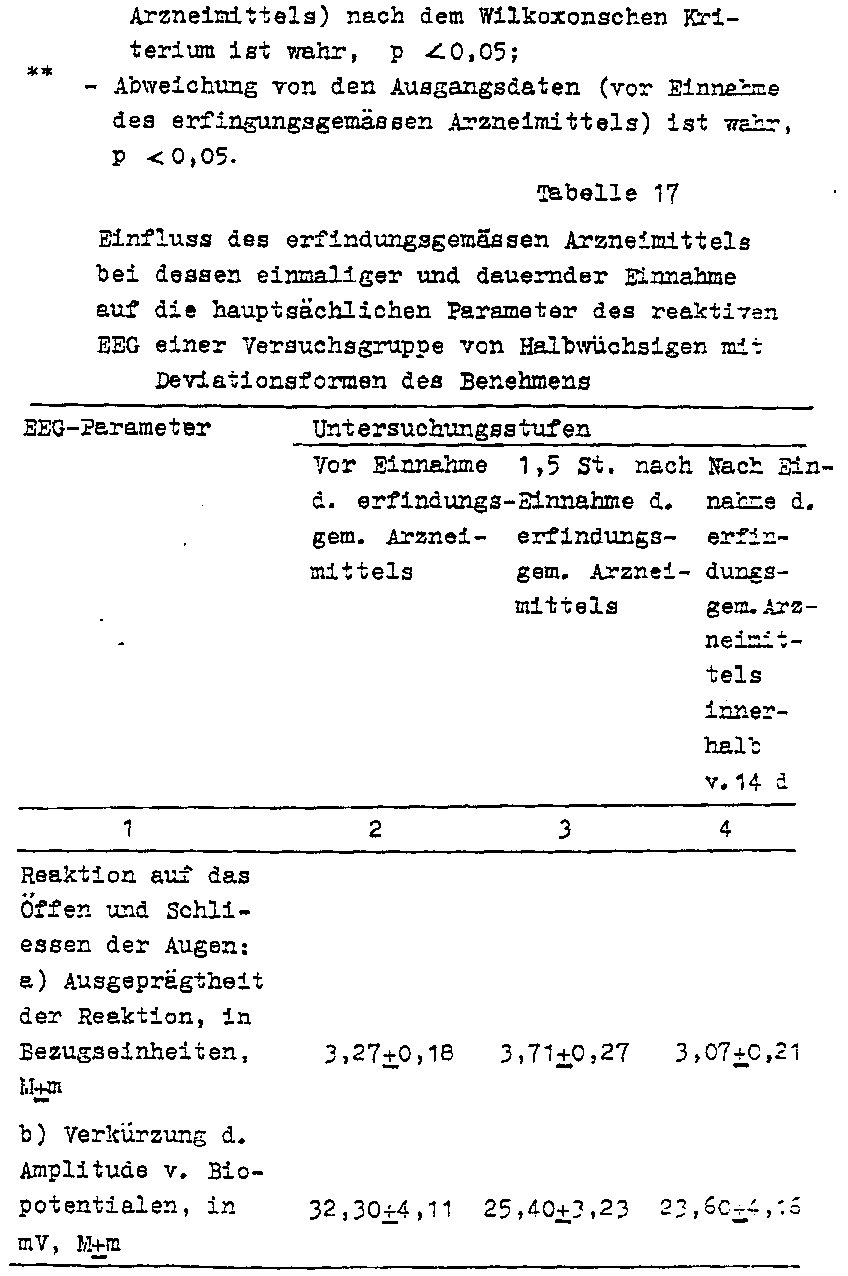

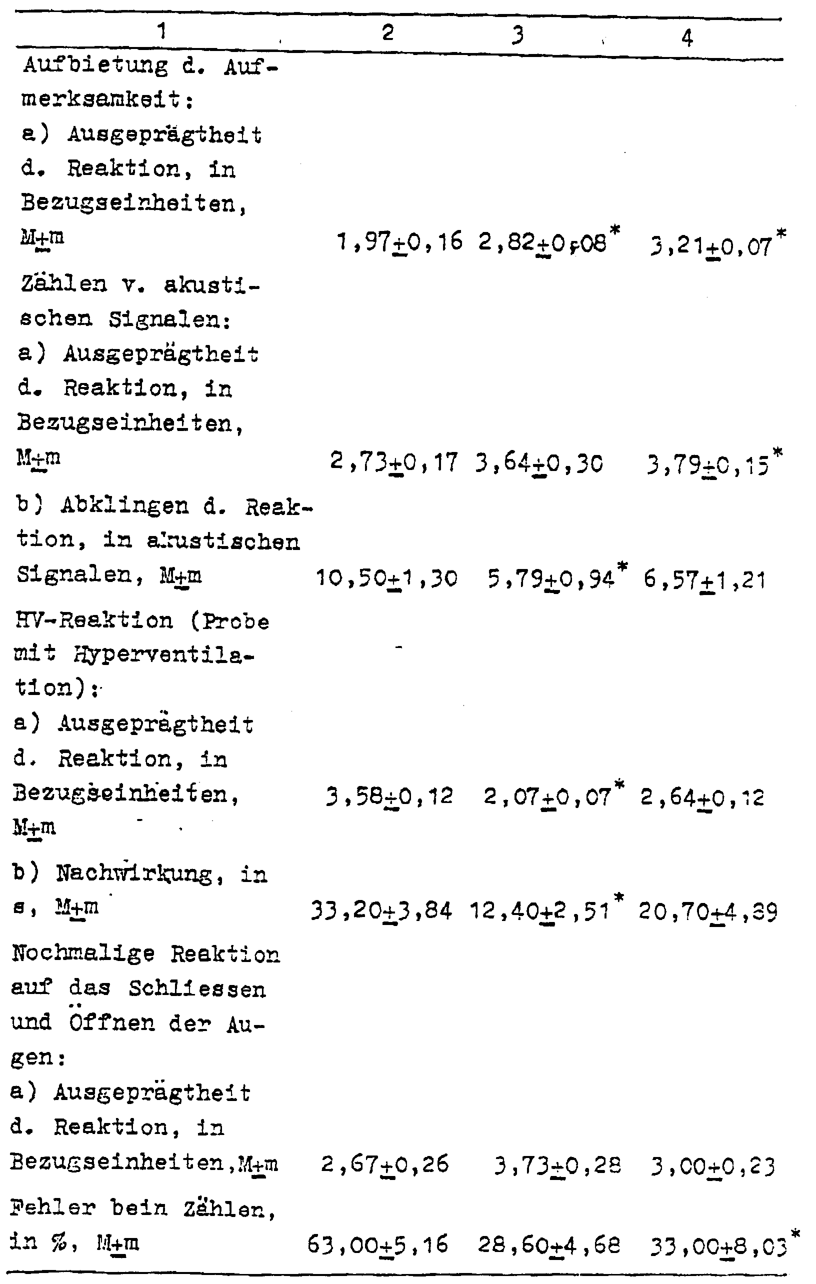

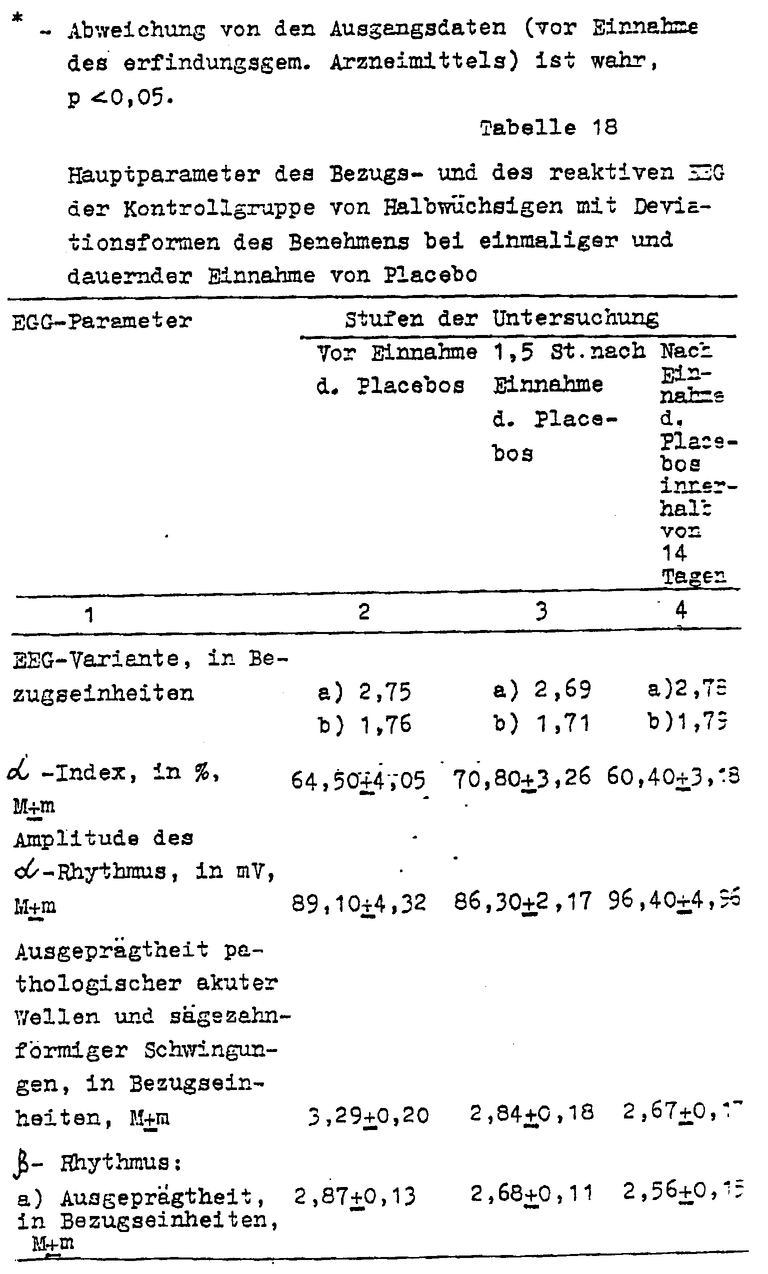

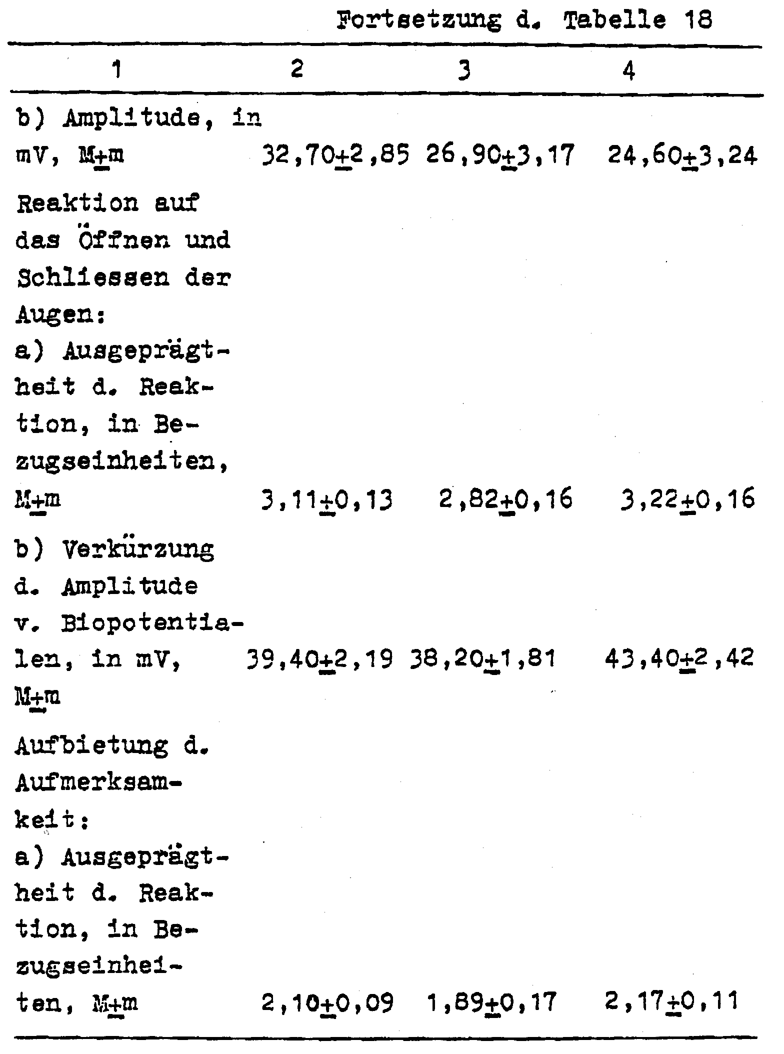

## Patentansprüche

1. Verwendung der Aminosäure Glycin oder deren pharmazeutisch zulässigen Salzen und einem pharmazeutischen Träger zur Herstellung von Arzneimitteln mit Antistress-, Stressschutz- und nootroper Wirkung für sublingualen Gebrauch.

2. Verwendung nach Anspruch 1 in Form von Tabletten, **dadurch gekennzeichnet**, daß das Arzneimittel Glycin in einer Menge zwischen 0,1 und 0,2 g pro Tablette aufweist.

3. Verwendung nach Anspruch 1 bzw. 2, **dadurch gekennzeichnet**, daß das Arzneimittel als pharmazeutischen Träger Methylcellulose in einer Menge von 0,5 bis 2 Gew.-% enthält.

## Claims

1. Use of the amino acid glycine or pharmaceutically acceptable salts thereof and a pharmaceutical vehicle for the preparation of drugs with an anti-stress, stress-prevention and nootropic action for sublingual use.

2. Use as claimed in claim 1 in the form of tablets, **characterised in that** the drug has glycine in a quantity of between 0.1 and 0.2 g per tablet.

3. Use as claimed in claim 1 or claim 2, **characterised in that** the drug contains as pharmaceutical vehicle methylcellulose in a quantity of 0.5 to 2 % by weight.

## Revendications

1. Utilisation de l'acide aminé glycine ou de ses sels pharmaceutiquement acceptables et d'un véhicule pharmaceutique pour la fabrication d'un médicament à action, anti-stress, de prévention du stress et nootrope, pour l'administration sublinguale.

2. Utilisation selon la revendication 1, sous forme de comprimés, caractérisée en ce que la glycine, servant de substance médicamenteuse, est présente en une quantité de 0,1 à 0,2 g par comprimé.

3. utilisation selon les revendications 1 ou 2, caractérisée en ce que le médicament renferme, en tant que véhicule pharmaceutique, de la méthylcellulose en une quantité de 0,5 à 2% en poids.
